# FASCICULE DE BREVET EUROPEEN

(11) **EP 0 655 136 B1**
(45) Date de publication et mention de la délivrance du brevet: **06.05.1999**
(21) Numéro de dépôt: 94918907.0
(22) Date de dépôt: 10.06.1994
(51) Int. Cl.: G01N 33/547, G01N 33/543, G01N 33/74

(54) **DISPOSITIF POUR LE DOSAGE D'HAPTENES ET SON UTILISATION**
VORRICHTUNG ZUR ANALYSE VON HAPTENEN UND IHRE VERWENDUNG
HAPTEN ASSAY DEVICE AND USE THEREOF

(30) Priorité: 11.06.1993 FR 9307093
(43) Date de publication de la demande: 31.05.1995
(73) Titulaire: BIO MERIEUX, 69280 Marcy l'Etoile (FR)
(72) Inventeur: CROS, Philippe, F-69005 Lyon (FR); KURFURST, Robin, F-69007 Lyon (FR); BATTAIL, Nicole, F-69002 Lyon (FR); PIGA, Nadia, F-69130 Ecully (FR)
(74) Mandataire: Tonnellier, Jean-Claude
(86) Numéro de dépôt international: FR9400689
(87) Numéro de publication internationale: WO9429723

(56) Documents cités:
- EP-A- 0 089 806
- EP-A- 0 205 643
- EP-A- 0 310 251
- EP-A- 0 524 864
- WO-A-83/03306
- WO-A-91/19812
- BIOCONJUGATE CHEM., vol.2, 1991, WASHINGTON, DC pages 217 - 225 REED ET AL. 'Acridine- and cholesterol-derivatized solid supports ....' cité dans la demande

## Description

La présente invention a pour objet un dispositif pour le dosage d'haptènes ou d'anticorps anti-haptène, et son utilisation.

Les haptènes sont de petites molécules non immunogènes, c'est à dire incapables par elles mêmes de promouvoir une réaction immunitaire par formation d'anticorps, mais capables d'être reconnues par des anticorps obtenus par immunisation d'animaux dans des conditions connues, en particulier par immunisation avec un conjugué haptène-protéine.

Diverses techniques ont été proposées pour le dosage d'haptènes dans un échantillon, parmi lesquelles les techniques dites de compétition. Par exemple, des auteurs ont décrit une technique de compétition en phase antigène, dans laquelle un haptène, qui est identique à l'haptène à doser, et qui est fixé sur une phase solide, entre en compétition avec l'haptène de l'échantillon pour sa liaison avec un anticorps spécifique marqué introduit en quantité définie. Cette technique a été décrite par exemple pour le dosage de la thyroxine, du cortisol et de la testostérone (voir notamment les demandes de brevet EP 089806 et WO 83/03306).

Toutefois, en raison de leur petite taille, il est impossible d'adsorber directement les haptènes sur une phase solide constituée d'un matériau polymère tout en conservant la disponibilité vis-à-vis du site de liaison d'un anticorps spécifique. Ainsi dans les techniques conventionnelles (voir notamment Clinica Chemica Acta, 162 (1987) 199-206 Elsevier) l'haptène est greffé sur un ligand protéique ou polypeptidique en vue de son immobilisation sur une phase solide. Cette technique présente cependant un inconvénient important inhérent à la non-immunogénicité des haptènes. En effet, la production d'anticorps anti-haptènes nécessite l'immunisation d'un animal contre des haptènes couplés à des protéines ou à des polypeptides, de façon à les rendre immunogènes, et les anticorps anti-haptènes produits en réponse peuvent interférer par réactions croisées avec des protéines ou polypeptides auxquels est couplé l'haptène pour sa fixation sur une phase solide. Par ailleurs, l'accès de l'anticorps à l'haptène fixé sur le support peut être gêné par des phénomènes de réorganisation intramoléculaire des protéines ou des polypeptides dans certaines conditions physico-chimiques, ce qui peut introduire des erreurs significatives dans la précision d'un test.

Aussi, la présente invention a pour objet un support approprié pour le dosage des haptènes qui pallie les inconvénients sus-mentionnés et qui permet en outre de contrôler le nombre d'haptènes immobilisés sur la phase solide et donc de réaliser un véritable dosage quantitatif. En outre, l'invention fournit un support permettant une meilleure orientation des haptènes immobilisés et donc une accessibilité accrue de ceux-ci pour leur liaison avec des anticorps lors des dosages.

On a en effet découvert que, de façon surprenante, il est possible de remédier aux inconvénients précités en utilisant comme intermédiaire de fixation un fragment d'acide nucléique.

L'invention a donc pour objet un dispositif pour le dosage d'un haptène, comprenant un support solide et un réactif lié de façon covalente à un ligand pour former un conjugué permettant d'immobiliser ledit réactif sur ledit support solide, ledit réactif étant reconnu par des anticorps capables de reconnaître ledit haptène, caractérisé par le fait que ledit ligand est un fragment d'acide nucléique.

On comprend que le réactif peut être soit l'haptène à doser soit un composé ayant une analogie immunologique suffisante avec ledit haptène pour que des anticorps reconnaissant l'un reconnaissent également l'autre.

Le terme haptène désigne la molécule à doser ou un analogue capable de réagir avec un anticorps spécifique de la molécule à doser.

Par exemple, les haptènes peuvent être des peptides, des peptides glycosylés, des métabolites, des vitamines, des hormones, des prostaglandines, des toxines, ou divers médicaments.

L'haptène peut être choisi par exemple parmi :
- un peptide glycosylé tel que la séquence N-terminale de la sous-unité bêta de l'hémoglobine humaine ;
- les hormones thyroïdiennes notamment la thyroxine, la triiodothyronine ; les hormones stéroïdiennes notamment les oestrogènes tels que l'estriol et l'estradiol, les androgènes tels que la testostérone, les progestatifs tels que la progestérone, les glucocorticoïdes tels que la 11-désoxycorticostérone et le cortisol; les catécholamines telles que l'adrénaline, la noradrénaline, la dopamine ;
- les vitamines A, B comme la B12, C, D, E et K, l'acide folique, la biotine, la thiamine ;
- les nucléosides et nucléotides comme l'adénosine di- ou triphosphate (ADP et ATP), la flavine mononucléotide (FMN), la nicotinamide adénine dinucléotide (NAD), son dérivé phosphate (NADP), la thymidine, la guanosine et l'adénosine ;
- des médicaments tels que la digoxine, la digitoxigénine, la digitoxine, la digoxigénine ; les antibiotiques notamment les aminoglycosides, la gentamicine, la tobramycine, l'amikacine, la sisomicine, la kanamycine, la netilmicine, la pénicilline, la tétracycline, la terramycine (nom commercial) ; ou le phénobarbital, la primidone, l'éthosuximide, la carbamazépine, le valproate, la théophylline, la caféine, le propanolol, le procainamide, la quinidine, l'amitriptyline, la désipramine, le disopyramide, etc. ;
- les toxines comme l'alphatoxine, la toxine du choléra, l'entérotoxine B du staphylocoque et analogues.

Le produit résultant du couplage covalent ligand-réactif est appelé ci-après "conjugué".

Selon un mode de réalisation de l'invention, le conjugué est immobilisé sur le support solide directement par adsorption passive ou fixation par covalence du fragment d'acide nucléique du conjugué.

Selon un autre mode de réalisation, le conjugué est immobilisé sur le support solide par l'intermédiaire d'un anti-conjugué, lui-même immobilisé ou immobilisable sur le support solide par covalence ou par adsorption passive, l'anti-conjugué étant un agent permettant l'immobilisation du conjugué par interaction avec le fragment d'acide nucléique du conjugué ou par interaction avec l'haptène et avec un groupement présent à la jonction du fragment d'acide nucléique et de l'haptène.

L'anti-conjugué peut être par exemple un second fragment d'acide nucléique capable de s'hybrider avec le fragment d'acide nucléique du conjugué.

Dans un autre mode de réalisation de l'invention, l'anti-conjugué est un anticorps ou un fragment d'anticorps dirigé contre le fragment d'acide nucléique du conjugué.

Selon un autre mode de réalisation de l'invention, l'anti-conjugué est une protéine susceptible de se lier directement ou indirectement au fragment d'acide nucléique du conjugué dans des conditions prédéterminées.

Le fragment d'acide nucléique est choisi notamment dans le groupe consistant en un fragment d'ADN ou d'ARN naturel ou de synthèse, ou un désoxyribonucléotide ou un ribonucléotide, ou un désoxyribonucléoside ou un ribonucléoside. Ce fragment d'acide nucléique peut être simple brin, double brin, ou partiellement double brin. De préférence, on utilise un fragment simple brin.

Le fragment d'acide nucléique est un oligodésoxyribonucléotide ou un oligoribonucléotide comprenant par exemple de 2 à 100 nucléotides, en particulier de 10 à 50 nucléotides.

Le fragment d'acide nucléique peut en outre présenter une ou plusieurs bases modifiées ou être composé uniquement de bases modifiées naturelles telles que la céto-6-purine, la xanthine, la méthyl-5-cytosine, l'amino-2-purine ou non naturelles telles que la thioguanine ou l'oxo-8-guanine. Le fragment d'acide nucléique peut présenter un ou plusieurs ou être composé uniquement de nucléosides modifiés sur la partie osidique tel que des carbonucléosides ou présenter des modifications au niveau du squelette phosphodiester tel que des groupements phosphorothioates. De même il peut présenter une ou plusieurs modifications ou être uniquement composé de nucléosides d'anomérie alpha ou bêta ou d'isomères de la série D ou L ; tous ces éléments pouvant être pris indépendamment ou en combinaison.

Le terme "support solide" tel qu'utilisé ici inclut tous les matériaux polymères sur lesquels peut être immobilisé un fragment d'acide nucléique pour une utilisation dans des tests diagnostiques. Des matériaux naturels, de synthèse, modifiés ou non chimiquement peuvent être utilisés comme support solide, notamment des polysaccharides tels que les matériaux de cellulose, par exemple du papier, des dérivés de cellulose tels que l'acétate de cellulose et la nitrocellulose ; des polymères tels que le polychlorure de vinyle, le polyéthylène, les polystyrènes, les polyamides, les polyacrylates, les polyuréthanes, les polycarbonates, les polytétrachloroéthylènes, ou des copolymères tels que les polymères chlorure de vinyle- propylène, chlorure de vinyle- acétate de vinyle, les copolymères à base de styrène ou de dérivés substitués du styrène, et des fibres synthétiques telles que le nylon.

Le matériau polymère est notamment un copolymère chlorure de vinyle/acétate de vinyle, chlorure de vinyle/propylène, ou un copolymère de styrène tel qu'un copolymère butadiène-styrène ; ou un copolymère butadiène-styrène en mélange avec un ou plusieurs polymères ou copolymères choisi parmi le polystyrène, les copolymères styrène-acrylonitrile ou styrène-méthylmétacrylate de méthyle, les polypropylènes, les polycarbonates ou analogues. Avantageusement, le support solide de la présente invention est un polystyrène ou un copolymère à base de styrène comprenant entre environ 10 et 90 % en poids de motifs styrène.

Le dispositif selon l'invention peut se présenter notamment sous la forme d'une plaque de microtitration, d'une feuille, d'un cône, d'un tube, d'un puits, de billes, de particules, de latex ou analogue.

Le terme "latex" désigne une dispersion aqueuse colloïdale d'un polymère quelconque insoluble dans l'eau.

La présente invention nécessite la préparation de fragments d'acide nucléique conjugués à une ou plusieurs molécules d'haptène par l'intermédiaire d'une liaison covalente. Cette liaison covalente peut être établie en suivant des approches directes ou indirectes. Ces techniques permettent d'obtenir une molécule composite modifiée de façon parfaitement connue quant à la position et au degré de substitution.

Par approche directe, on entend le couplage en synthèse automatique de l'haptène additionné comme un nucléotide au cours de la synthèse c'est-à-dire en position 5' et/ou 3' ou en toute autre position dans la chaîne. Ceci nécessite la préparation d'un dérivé utilisable en synthèse automatique, par exemple, un dérivé phosphoramidite, H-phosphonate ou phosphotriester de l'haptène ou d'un nucléotide substitué par l'haptène sur la base ou sur la partie osidique. De même l'haptène peut être introduit au cours de la synthèse sur les phosphores internucléotidiques par phosphoramidation oxydative.

Deux voies sont envisagées pour les approches indirectes :

La première nécessite, de façon connue en soi, l'introduction sur les deux partenaires, c'est à dire sur le fragment d'acide nucléique et sur l'haptène, respectivement, des fonctions réactives l'une par rapport à l'autre. A titre d'exemple une fonction amine primaire portée par l'un des partenaires réagira avec l'ester N-hydroxysuccinimique de l'autre partenaire, de même une fonction thiol réagira sur un groupement maléimide, pyridyldisulfure ou halogénoalkyle et un phosphorothioate réagira avec un halogénoalkyle.

La deuxième voie implique l'introduction sur le fragment d'acide nucléique et sur l'haptène de fonctions ayant des réactivités spécifiques, mais ces fonctions ne sont pas réactives directement l'une par rapport à l'autre. La liaison s'établit par l'intermédiaire d'un agent de couplage.

Le terme agent de couplage tel qu'utilisé ici désigne une molécule contenant au moins deux extrémités ou groupements réactifs de même nature ou de nature différente (agent de couplage homobifonctionnel ou hétérobifonctionnel).

A titre d'exemple, une fonction amine primaire et une fonction acide carboxylique peuvent former une liaison amide en présence d'un carbodiimide, deux fonctions amines primaires seront couplées en présence de phénylène-1,4-diisothiocyanate ou de disuccinimidylsubérate.

Les fonctions réactives introduites sur le fragment d'acide nucléique peuvent être choisies notamment parmi les groupements amine, hydrazinoyle, hydrazonoyle, iminoyle, azidoyle, triazenoyle, triazanoyle, amide éventuellement substituée, semicarbazonoyle, aldéhydyle, alkylthiol, arylthiol,carbamoyle, carboxyle, cyanoyle, halogénoalkyle, hydroxyle, N-hydroxysuccinimidyle, maléimide, phosphate, phosphorodithioate, phosphite, phosphonate, phosphorothioate, pyridyldisulfure, sulfamoyle, thiophosphate.

Lesdites fonctions réactives introduites (ou éventuellement déjà présentes) sur l'haptène peuvent être choisies à titre d'exemple parmi les groupements amine, hydrazinoyle, hydrazonoyle, iminoyle, azidoyle, triazenoyle, triazanoyle, amide éventuellement substituée, semicarbazonoyle, aldéhydyle, alkylthiol, arylthiol,carbamoyle, carboxyle, cyanoyle, halogénoalkyle, hydroxyle, N-hydroxysuccinimidyle, maléimide, phosphate, phosphorodithioate, phosphite, phosphonate, phosphorothioate, pyridyldisulfure, sulfamoyle, thiophosphate.

L'introduction de ces fonctions réactives (et par conséquent le greffage ultérieur de l'haptène) sur les fragments d'acide nucléique peut s'effectuer en n'importe quelle position de la chaîne en utilisant par exemple des réactifs synthétisés selon des méthodes connues ou disponibles dans le commerce. A titre d'exemple les modifications peuvent être réalisées en position 5' par emploi d'un réactif type Aminolink II (Applied Biosystems réf. 400808) ; en position 3' par utilisation d'une phase solide telle que celle vendue par la société Clontech Lab Inc sous la référence 5221, ou celle décrite par Reed et al. (1991) Bioconjugate Chem., 2,217-225 ; sur les bases nucléiques (Glen Research réf. 101039) ; sur la partie osidique par modification de la partie osidique en utilisant par exemple un ribofuranose substitué en position 2' à la place d'un désoxy-2'-ribofuranose (Yamana et al. (1991) Tetrahedron Lett., 32, 6347-6350), ou en substituant un nucléoside par un composé alkylamine (Clontech Lab Inc ref 5203) ou encore par substitution d'un phosphate internucléotique selon des protocoles décrits par Froehler et al., (1988) Nucl. Acids Res., 16, 11,4831-4839.; Conway et McLaughlin (1991) Bioconjugate Chem., 2, 452-457 ; Letsinger et al. (1989) Proc. Natl. Acad. Sci. USA, 86,6553-6556.

De même, si nécessaire la modification de l'haptène pour l'introduction d'une fonction réactive peut être réalisée en toute position appropriée de la molécule d'haptène. Un exemple de modification est donné dans "Preparation of antigenic steroid-protein conjugate", F. Kohen et al., dans Steroid immunoassay, Proceedings of the fifth tenovus workshop, Cardiff, Avril 1974, ed. EHD Cameron, SH. Hillier, K. Griffiths.

La fixation du conjugué fragment d'acide nucléique-haptène sur la phase solide peut être réalisée de manière directe ou indirecte.

De manière directe, deux approches sont possibles : soit par adsorption du conjugué sur le support solide, c'est à dire par des liaisons non covalentes (principalement de type hydrogène, Van der Walls ou ionique), soit par établissement de liaisons covalentes entre le conjugué et le support.

De manière indirecte, on peut fixer préalablement (par adsorption ou covalence) sur le support solide un composé anti-conjugué capable d'interagir avec le fragment d'acide nucléique du conjugué de façon à immobiliser l'ensemble sur le support solide.

A titre d'exemple, ce composé anti-conjugué peut être : un anticorps anti-fragment d'acide nucléique tel que décrit par P. CROS et al. (monoclonal antibodies to alpha deoxyribonucleotides, (1993), NAMA conférence, poster 4-37) ; un fragment d'acide nucléique ayant une séquence au moins partiellement complémentaire du fragment d'acide nucléique du conjugué ; une protéine fixant spécifiquement le fragment d'acide nucléique simple brin du conjugué telle que la protéine de liaison simple brin de E.coli ou la protéine codée par le gène 32 du bactériophage T4 ; un système ligand-récepteur, par exemple en greffant sur le fragment d'acide nucléique une molécule telle qu'une vitamine et en immobilisant sur la phase solide le récepteur correspondant (par exemple le système biotine- streptavidine) ; une protéine se fixant sur une séquence spécifique couramment appelée promoteur, comme les ARN polymérases et notamment les ARN polymérases phagiques telles que les T7, T3, SP6 ARN polymérases.

Le dispositif selon l'invention est utilisable pour effectuer un dosage d'haptènes par compétition dans un échantillon, notamment un fluide biologique, dans lequel le réactif immobilisé entre en compétition avec l'haptène de l'échantillon pour sa liaison à un anticorps spécifique introduit dans des proportions prédéterminées. On révèle la présence de l'anticorps spécifique fixé sur le support solide par les méthodes usuelles. Par exemple l'anticorps spécifique est marqué par tout marqueur approprié, ou bien le complexe immun (haptène immobilisé/anticorps anti-haptène) fixé sur le support solide, susceptible d'être formé, est révélé par l'utilisation d'un anticorps anti-complexe marqué par tout marqueur approprié. De préférence, l'anticorps anti-complexe est un anticorps dirigé contre l'anticorps du complexe formé.

L'invention a également pour objet un procédé de dosage d'un haptène dans un échantillon liquide caractérisé par le fait :
- que l'on ajoute audit échantillon une quantité prédéterminée d'anticorps capables de reconnaître ledit haptène ;
- que l'on met en contact l'échantillon avec le dispositif tel que décrit précédemment ;
- que l'on évalue la quantité d'anticorps fixé sur le support solide ;
- et que l'on en déduit, selon les méthodes connues la quantité d'haptènes présente dans l'échantillon.

Ce procédé est mis en oeuvre selon des techniques connues en soi. Bien entendu, les deux premières étapes peuvent être réalisées dans un ordre quelconque. On évalue la quantité d'anticorps fixé après un temps d'incubation approprié.

On peut par exemple déterminer la quantité d'haptène à l'aide d'une courbe-étalon, obtenue par étalonnage du dispositif à l'aide d'échantillons contenant diverses quantités connues de l'haptène, et permettant d'établir la corrélation entre la quantité d'anticorps fixé et la quantité d'haptènes correspondante.

Le dispositif de l'invention peut également servir au dosage d'anticorps anti-haptène dans un échantillon. Pour cela, on peut, selon un mode de réalisation particulier, mettre l'échantillon en contact avec le dispositif, puis révéler, selon les méthodes connues, la présence éventuelle d'anticorps anti-haptène fixés. On peut effectuer la révélation par exemple à l'aide d'anticorps marqués dirigés contre le fragment Fc des anticorps de l'espèce dont sont issus les anticorps anti-haptène recherchés. Selon un autre mode de réalisation, on peut opérer par compétition en ajoutant à l'échantillon une quantité connue d'anticorps anti-haptène marqués et en déterminant la quantité d'anticorps marqués fixés. Par comparaison avec une courbe d'étalonnage établie préalablement, on peut en déduire la quantité d'anticorps anti-haptène présents à l'origine dans l'échantillon.

On va maintenant donner, à titre d'illustration, des exemples de préparation du dispositif selon l'invention ainsi que des exemples d'utilisation dudit support. Dans ces exemples, l'expression "speed vac" désigne un concentrateur-lyophilisateur rotatif.

### EXEMPLE 1 : Synthèse d'oligonucleotides pour le couplage sur haptène :

Les oligonucléotides sont synthétisés sur un appareil automatique 394 de la société APPLIED BIOSYSTEMS en utilisant la chimie des phosphoramidites selon le protocole du constructeur. Pour permettre le couplage de l'oligonucléotide à un haptène sur une position bien déterminée, des fonctions réactives sont introduites sur les oligonucléotides par l'intermédiaire de bras de liaison compatibles avec la synthèse automatique. Les bras utilisés dans la présente invention et donnés ici à titre d'exemple sont décrits dans le tableau 1.

Le bras phosphoramidite référencé 1 est additionné à l'extrémité 5' de l'oligonucléotide selon le protocole standard de la synthèse automatique. La fonction réactive se trouve à l'extrémité 5' de l'oligonucléotide.

Dans le cas du composé portant la référence 2, la fonction réactive est portée par la base thymine sur la position 5. Ce composé peut être introduit à n'importe quelle position de l'oligonucléotide à l'exception de l'extrémité 3'.

Dans le cas du composé portant la référence 3, la synthèse automatique démarre par la silice greffée par le bras selon le protocole standard. La fonction réactive se trouve à l'extrémité 3' de l'oligonucléotide.

Après déprotection une nuit à 55°C dans NH₄OH 33 %, suivie d'une précipitation dans de l'éthanol à -20°C, les oligonucléotides sont séchés sous vide et repris dans 1 ml d'eau.

Dans tous les cas, les oligonucléotides modifiés sont purifiés par chromatographie liquide haute pression (CLHP) en phase inverse sur une colonne Brownlee RP18 (10 mm - 25 cm).

### Conditions :

Débit 4,6 ml/min,
- Gradient :: 10 % à 35 % de tampon B en 30 min,
35 % à 100 % de tampon B en 3 min.
Les caractéristiques des tampons A et B sont les suivantes :
- Tampon A :: 0.1 molaire Triethylammoniumacétate (TEAA) pH = 7,00.
- Tampon B :: 50 % Tampon A + 50 % CH3CN.

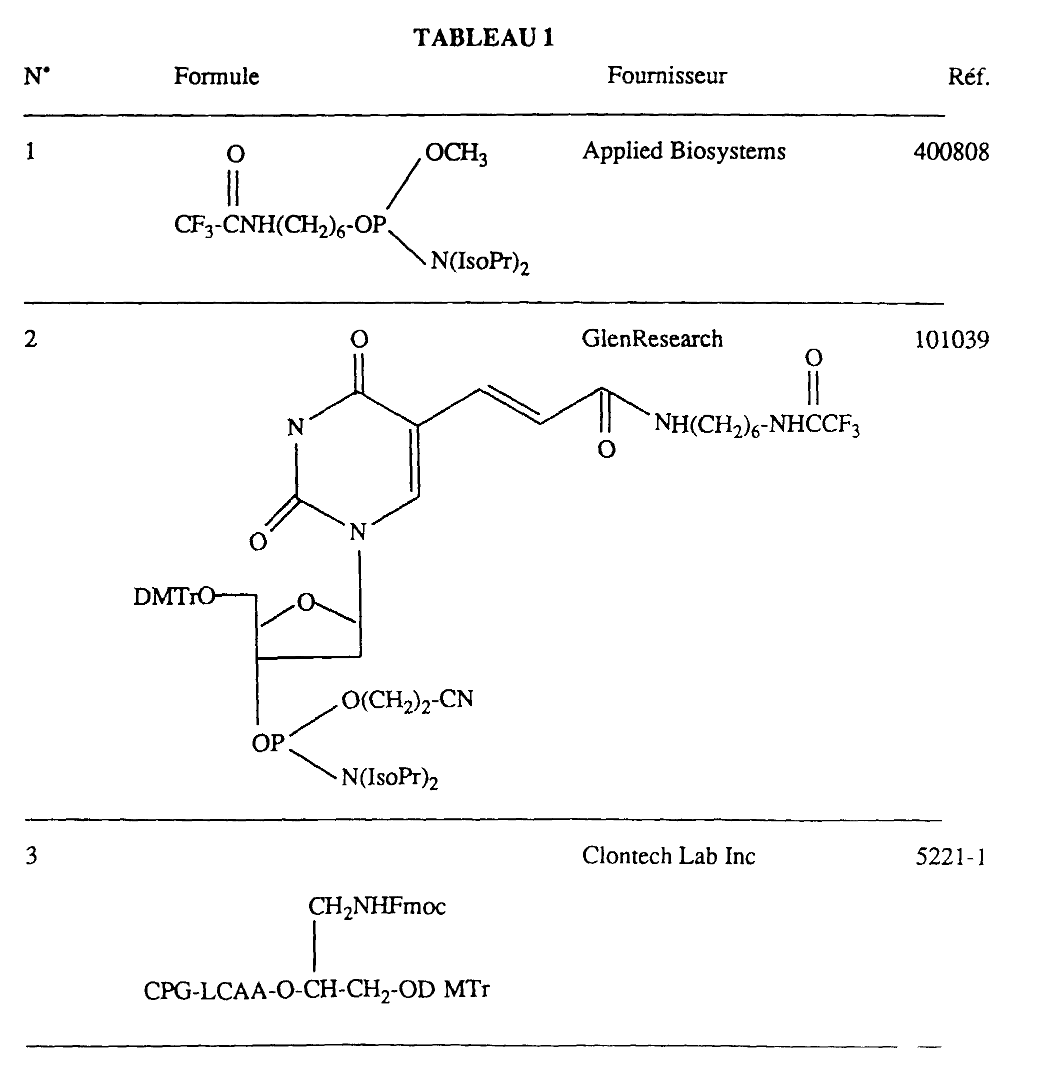

DMTr = diméthoxytrityle
Fmoc = fluorenyl-9-méthoxycarbonyle
CPG = billes de verre à porosité contrôlée
LCAA = longue chaine alkyle amine (bras espaceur)
Les oligonucléotides modifiés par les bras du tableau 1 et utilisés dans la présente invention sont décrits dans le tableau 2.

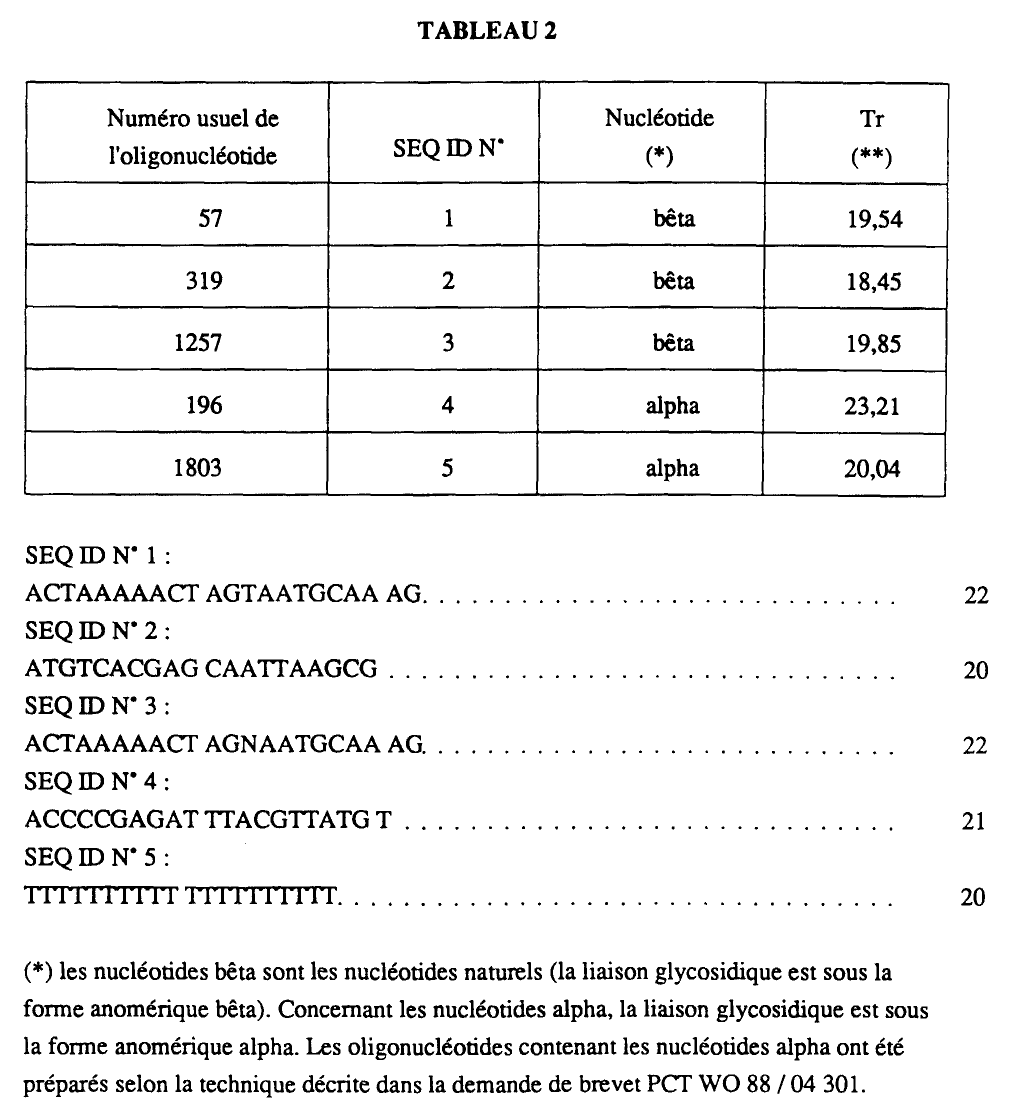

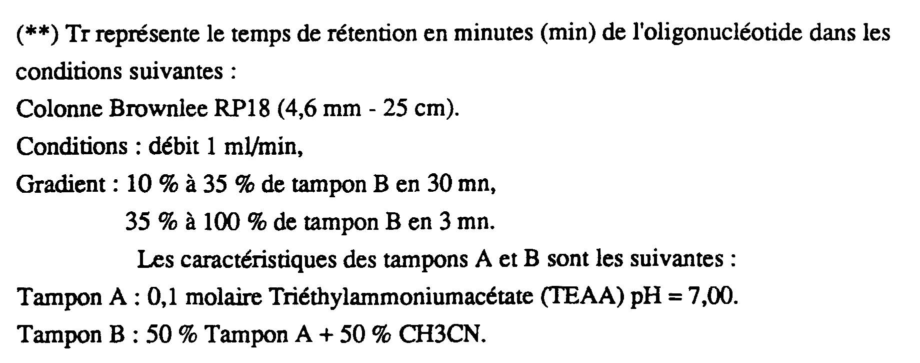

### Couplage d'un haptène sur un oligonucléotide :

Les haptènes utilisés dans la présente invention et donnés ici à titre d'exemple sont décrits dans le tableau 3.

**TABLEAU 3**

| HAPTENE à DOSER | HAPTENE ACTIVE UTILISE LE COUPLAGE | FOURNISSEUR | REFERENCE |
|---|---|---|---|
| ESTRADIOL | ESTRADIOL-6-CARBOXYMETHOXIME-N-HYDROXYSUCCINIMIDE ESTER | BOEHRINGER MANNHEIM | 05.21041 |
| TETRAIODO-THYRONINE T4 | N-BOC-THYROXINE-N-HYDROXY-SUCCINIMIDE ESTER | BOEHRINGER MANNHEIM | 05.21003 |
| TRIIODOTHYRONINE T3 | N-BOC-TRIIODOTHYRONINE-N-HYDROXYSUCCINIMIDE ESTER | BOEHRINGER MANNHEIM | 05.21002 |
| TESTOSTERONE | TESTOSTERONE-19-HEMISUCCINATE-N-HYDROXYSUCCINIMIDE ESTER (*) | BIOMERIEUX | / |

| | | | |
|---|---|---|---|
| (*) Dérivé de la 19-hydroxy testostérone. | | | |

L'haptène testostérone, fourni par BIOMERIEUX, est préparé selon la méthode décrite dans J. Steroid Biochem., 23(6a), pages 981-989, 1985 par A. WHITE et collaborateurs.

50 nmoles d'oligonucléotides sont séchées au speed vac et repris dans 20 µl de tampon borate de sodium 0,1 M, pH 9,3. 300 µl d'une solution d'haptène activé (5 mg/ml dans le DMF) sont ajoutés goutte à goutte puis laissés sous agitation 3 heures à 50°C. Après centrifugation, le surnageant séparé, le précipité résultant de la centrifugation repris dans 100 µl d'eau puis additionné au surnageant. Le mélange est traité avec 250 µl d'acétate d'ammonium 3M pH 5,2 puis 5 ml d'éthanol froid (-20°C). Après 30 minutes à-80°C et centrifugation, le culot est repris dans 500 µl d'eau.

Le conjugué oligonucléotide-haptène est purifié par CLHP dans les conditions suivantes :
Colonne BECKMAN ODS (10 mm- 25 cm).
- Conditions :: débit 4,6 ml/min,
- Gradient :: 20 % à 30 % de tampon B en 10 min,
30 % à 70 % de tampon B en 20 min.
Les caractéristiques des tampons A et B sont les suivantes :
- Tampon A :: 0,1 molaire Triéthylammoniumacétate (TEAA) pH = 7,00.
- Tampon B :: 50 % Tampon A + 50 % CH₃ CN.

Le pic correspondant au couplage est séché au speed vac puis repris dans 500 µl d'eau.

Les différents conjugués oligonucléotide-haptène préparés dans la présente invention sont résumés dans le tableau 4.

**TABLEAU 4**

| Conjugué oligonucléotide-haptène | Oligonucléotide | Haptène(*) | Tr (**) |
|---|---|---|---|
| A | 57 | ESTRADIOL | 16,18 |
| B | 57 | T3 | 27,44 |
| C | 57 | T4 | 25,79 |
| D | 57 | TESTOSTERONE | 27,96 (a) |
| E | 319 | ESTRADIOL | 14,50 |
| F | 1257 | T4 | 22,71 |
| G | 196 | ESTRADIOL | 15,65 |
| H | 1803 | ESTRADIOL | 15,45 |

| | | | |
|---|---|---|---|
| (*) voir le tableau 3 pour la formule exacte de l'haptène utilisé pour le couplage. | | | |
| (**) Tr est le temps de rétention du produit purifié dans les conditions suivantes : Colonne Brownlee RP 300 (4.6 mm x 100 mm). | | | |

- Débit :: 1,0 ml/min.
- Gradient :: 20 % à 30 % de tampon B en 10 min,
30 % à 70 % de tampon B en 20 min.
(a) pour le conjugué D le gradient est de 10 à 40% de B en 30 min.

Les caractéristiques des tampons A et B sont les suivantes :
- Tampon A :: 0.1 molaire Triéthylammoniumacétate (TEAA) pH = 7,00.
- Tampon B :: 50 % Tampon A + 50 % CH₃CN.

### EXEMPLE 2 : Utilisation d'un conjugué oligonucléotide-estradiol immobilisé par adsorption directe sur un support solide, pour la détermination d'estradiol par technique immunoenzymatique de type compétition.

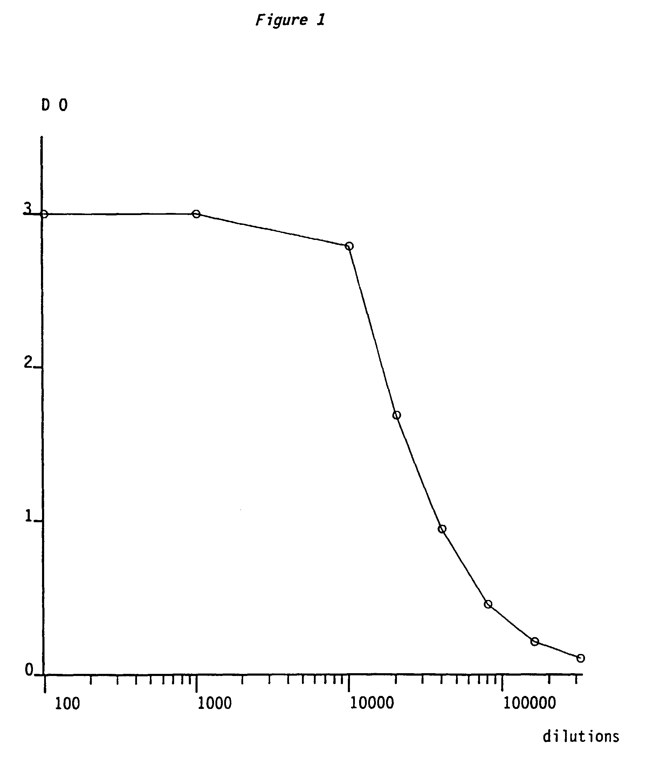

Un anticorps monoclonal anti-estradiol (BIOMERIEUX ref. 10G6E9H4C1D6) obtenu par la technique classique décrite par G. Köhler et C. Milstein (Nature 256. 495-497 (1975)) purifié à partir de liquides d'ascites par chromatographie de type échangeuse d'ions ABx (Baker-72 69 00), puis conjugué à de la peroxydase (commercialisée par la société FORDRAS) selon la technique décrite en 1974 par Nakané P.K. et Kawoi A. (J. Histochem. Cytochem, 22 : 1084) est utilisé à une concentration déterminée non saturante établie à partir d'une courbe de titrage. La courbe de titrage est obtenue comme suit : dans les puits d'une plaque de polystyrène Maxisorb NUNC (commercialisée par la société Polylabo Paul Block sous la référence 4-39454), on immobilise le conjugué A obtenu comme décrit dans l'exemple 1 à raison de 100 µl par cupule et à une concentration de 0,15 µM dans du tampon PBS x 3 (NaCl 0,45 M ; phosphate de sodium 0,15 M ; pH 7,0). Cette plaque est incubée une nuit à 22°C ou une heure à une température de 37°C. La plaque est lavée trois fois par 300 µl de tampon PBS-Tween 0,05 % (NaCl 0,15 M; phosphate de sodium 0,05 M ; pH 7,0 ; Tween 20 à une concentration finale de 0,05 % (Tween : nom commercial)). Les sites dans la plaque non occupés par le conjugué oligonucléotide-haptène A sont saturés par l'addition de 100 µl d'une solution de PBS-lait à 1 % en concentration finale (NaCl 0,15 M; phosphate de sodium 0,05 M ; pH 7,0 ; lait écrémé Régilait 1 %). La plaque est incubée pendant une heure à 37°C, puis rincée 3 fois par 300 µl de tampon PBS-Tween 0,05 %. Dans chaque puits de la plaque sont ajoutés 100 µl de dilutions successives de l'anticorps anti-estradiol marqué en tampon PBS, et incubés pendant 15 minutes sous agitation à 22°C. La plaque est rincée trois fois avec du tampon PBS-Tween 0,05 %, puis une fois avec de l'eau distillée. 100 µl de substrat OPD (Ortho-Phénylène-Diamine, commercialisé par la société Cambridge Medical Biotechnology sous la référence 456) à la concentration de 4 mg/ml dans un tampon OPD (acide citrique 0,05 M ; NaH₂PO₄ 0,1 M ; pH 4,93) auquel est ajouté extemporanément H₂O₂ à 30 volumes dilué au 1/1000, sont introduits dans les puits de la plaque. La réaction enzymatique est bloquée par addition de 100 µl d'H₂SO₄ 1N après une incubation de 30 minutes à 37°C. La lecture est effectuée à 492 nm sur un lecteur Biowhittaker Microplate Reader 2001. La courbe de titrage obtenue est représentée à la figure 1 ; chaque point représente la moyenne de deux valeurs moins la valeur de la fixation non spécifique. A partir de cette courbe de titrage, l'anticorps anti-estradiol marqué, qui sera utilisé ultérieurement, est choisi à une dilution déterminée correspondant à une activité enzymatique non saturante, telle que donnant une D.O. de 1,5 à 492 nm.

La détermination de la molécule d'estradiol à doser est effectuée selon le même protocole que décrit précédemment, à l'exception de l'étape d'addition de l'anticorps anti-estradiol marqué. A cette étape, 50 µl d'anticorps anti-estradiol à la dilution précédemment déterminée sont préalablement mélangés à 50 µl d'une solution choisie parmi les solutions à concentration croissante d'estradiol. Les résultats sont représentés dans le tableau 5 ci-après.

**TABLEAU 5**

| Concentration d'estradiol pg/ml (*) | D.O.492nm (**) |
|---|---|
| 0 | 1,441 |
| 0,1 | 1,379 |
| 1 | 1,416 |
| 10 | 1,254 |
| 100 | 0,274 |
| 1000 | 0,019 |

| | |
|---|---|
| * Les échantillons contenant les différentes concentrations d'estradiol sont préparés par addition de quantités connues d'estradiol (17 bêta estradiol commercialisée par la société Sigma sous la référence E 1631) dans du tampon PBS. | |
| ** Chaque point représente la D.O. moyenne de deux valeurs moins la valeur de la fixation non spécifique (lecture 492 nm). | |

Les résultats de la gamme d'étalonnage présentés dans le tableau 5 montrent une bonne inhibition de la fixation de l'anticorps marqué lorsque la concentration en estradiol augmente.

### EXEMPLE 3 : Utilisation d'un conjugué oligonucléotide-testostérone immobilisé par adsorption directe sur un support solide, pour la détermination de testostérone par technique immunoenzymatique de type compétition.

Un sérum polyclonal de lapin anti-testostérone (commercialisé par la société Biospacific sous la référence 052B4089) est utilisé à une concentration déterminée non saturante établie à partir d'une courbe de titrage. La courbe de titrage est obtenue comme suit :

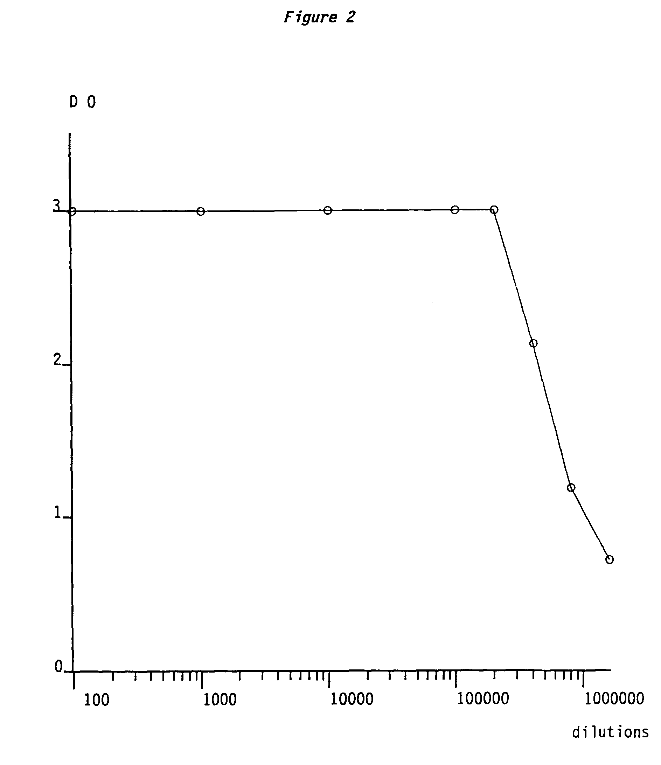

Dans les puits d'une plaque de polystyrène Maxisorb NUNC (commercialisée par la société Polylabo Paul Block sous la référence 4-39454) est immobilisé le conjugué oligonucléotide-haptène D obtenu comme décrit dans l'exemple 1 à raison de 100 µl par cupule et à une concentration de 0,15 µM dans du tampon PBS x 3 (NaCl 0,45 M ; Phosphate de sodium 0,15 M ; pH 7,0). Cette plaque est incubée une nuit à 22°C ou une heure à une température de 37°C. La plaque est lavée trois fois par 300 µl de tampon PBS-Tween 0,05 % (NaCl 0,15 M ; phosphate de sodium 0,05 M; pH 7,0 ; Tween 20 à une concentration finale de 0,05 % (Tween : nom commercial)). Les sites dans la plaque non occupés par le conjugué oligonucléotide-haptène D sont saturés par l'addition de 100 µl d'une solution de PBS-lait à 1 % en concentration finale (NaCl 0,15 M ; phosphate de sodium 0,05 M ; pH 7,0 ; lait écrémé Régilait 1 %). La plaque est incubée pendant une heure à 37°C, puis rincée 3 fois par 300 µl de tampon PBS-Tween 0,05 %. Dans chaque puits de la plaque sont ajoutés 100 µl de dilutions successives de sérum de lapin anti-testostérone en tampon PBS, et incubés pendant 15 minutes sous agitation à 22°C. La plaque est rincée trois fois par 300 µl de tampon PBS-Tween 0,05 %, puis 100 µl d'un anticorps polyclonal de chèvre anti-Fc d'IgG de lapin marqué à la phosphatase alcaline (commercialisé par la société Jackson Immunoresearch Laboratories sous la référence 111-055-046) dilué au 1/1000 en tampon PBS sont incorporés dans les puits de la plaque. Cette dernière est incubée pendant une heure à 37°C, puis est rincée trois fois avec du tampon PBS-Tween 0,05 %, puis une fois avec de l'eau distillée. 100 µl de substrat PNPP (paranitrophényl-phosphate commercialisé par la société Sigma sous la référence 104-O) à la concentration de 2 mg/ml dans du tampon diéthanolamine-HCl (diéthanolamine 1,29 M ; MgSO₄ 0,56 mM ; NaN₃ 0,38 mM ; HCl 0,012 N ; pH 9,8), sont ajoutés dans les puits de la plaque. La réaction enzymatique est bloquée par addition de 100 µl de NaOH 1 N après une incubation de 30 minutes à 37°C. La lecture est effectuée à 405 nm sur un lecteur Biowhittaker Microplate Reader 2001. La courbe de titrage obtenue est représentée à la figure 2 ; chaque point représente la moyenne de deux valeurs moins la valeur de la fixation non spécifique. A partir de cette courbe de titrage, l'anticorps anti-testostérone qui sera utilisé ultérieurement, est choisi à une dilution déterminée correspondant à une activité enzymatique non saturante, telle qu'une D.O. de 1,5 à 405 nm.

La détermination de la molécule de testostérone à doser est effectuée selon le même protocole que décrit précédemment, à l'exception de l'étape d'addition de l'anticorps anti-testostérone. A cette étape, 50 µl d'anticorps anti-testostérone à la dilution précédemment déterminée sont préalablement mélangés à 50 µl d'une solution choisie parmi des solutions à concentration croissante de testostérone. Les résultats sont représentés dans le tableau 6 ci-après.

**TABLEAU 6**

| Concentration de Testostérone pg/ml (*) | D.O. 405 nm (**) |
|---|---|
| 0 | 2,607 |
| 10 | 2,392 |
| 100 | 1,293 |
| 1000 | 0,467 |
| 10000 | 0,203 |

| | |
|---|---|
| * Les échantillons contenant les différentes concentrations de Testostérone sont préparés par addition de quantités connues de Testostérone (4-androstène-3-one-17-ol commercialisée par la société Sigma sous la référence T 1500) dans du tampon PBS. | |
| ** Chaque point représente la D.O. moyenne de deux valeurs moins la valeur de la fixation non spécifique (lecture 405 nm). | |

Les résultats de la gamme d'étalonnage présentés dans le tableau 6 montrent une bonne inhibition de la fixation de l'anticorps spécifique lorsque la concentration en testostérone augmente.

### EXEMPLE 4 : Utilisation d'un conjugué oligonucléotide-estradiol immobilisé par l'intermédiaire d'un oligonucléotide complémentaire sur un support solide pour la détermination d'estradiol par technique immunoenzymatique de type compétition.

L'anticorps monoclonal anti-estradiol utilisé est identique à celui décrit dans l'exemple 2 (BIOMERIEUX ref. 10G6E9H4C1D6).

La courbe de titration est obtenue conformément au protocole décrit dans l'exemple 2, à l'exception de la première étape qui se déroule comme suit :

Dans les puits d'une plaque de microtitration Maxisorb NUNC, est immobilisé un oligonucléotide d'une séquence complémentaire de la séquence nucléotidique de l'oligonucléotide 57 (séquence ID N°1) décrit dans l'exemple 1 à raison de 100 µl par cupule et à une concentration finale de 0,75 µM dans un tampon PBS x 3. Les plaques sont incubées une nuit à 22°C ou une heure à une température de 37°C. La plaque est lavée 3 fois par 300 µl de tampon PBS-Tween 0,05 %. Les sites de la plaque non occupés par cet oligonucléotide sont saturés par l'addition de 100 µl d'une solution de PBS-lait de concentration finale 1 %. La plaque est incubée 1 heure à 37°C, puis rincée 3 fois par 300 µl de tampon PBS-Tween 0,05 %. Dans chaque puits sont ajoutés 100 µl du conjugué oligonucléotide-haptène A obtenu comme décrit dans l'exemple 1 à une concentration de 0,15 µM, soit dans du tampon PBS 3 X, soit dans du tampon PEG (NaH₂ PO₄/Na₂ HPO₄ 0,1 M ; NaCl 0,5 M ; pH 7,0, Tween 20 : 0,05 % ; ADN de sperme de saumon 0,14 mg/ml ; PEG 4000 2 %) pendant 1 heure à 37°C. La plaque est lavée 3 fois par 300 µl de tampon PBS-Tween 0,05 %.

Le reste de l'expérimentation se déroule conformément au protocole décrit dans l'exemple 2.

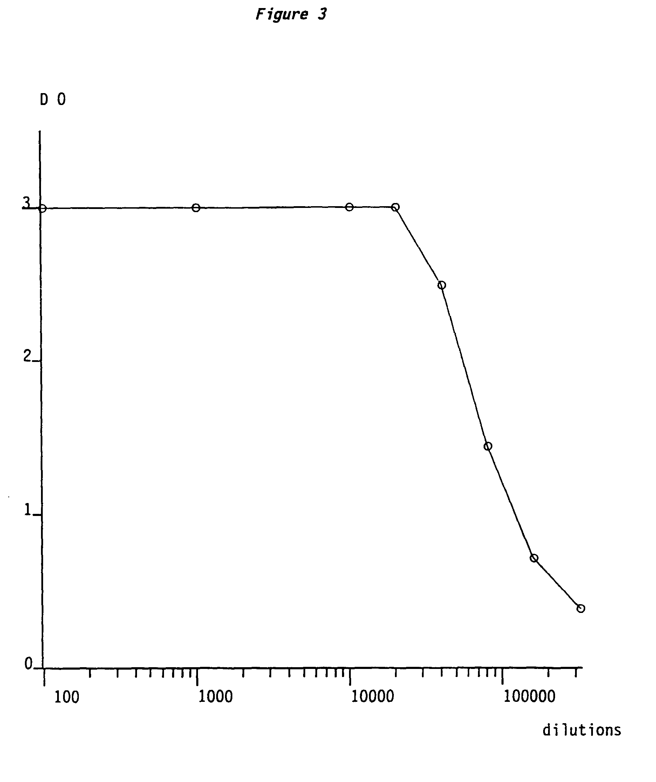

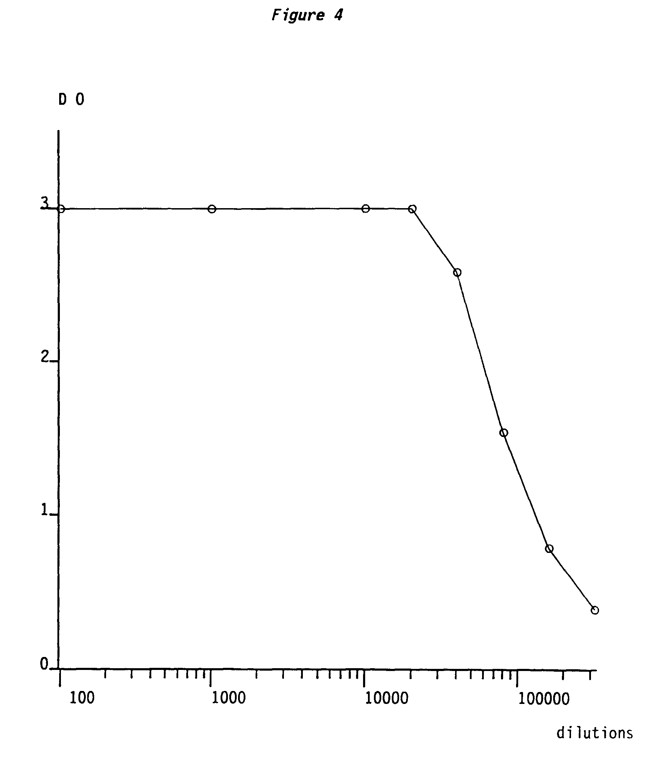

Les courbes de titration obtenues sont respectivement représentées dans les figures 3 (tampon PBS 3X) et 4 (tampon PEG). L'anticorps marqué, qui sera utilisé ultérieurement, est choisi à une dilution déterminée correspondant à une activité enzymatique non saturante, telle que donnant une D.O. de 1,5 lue à 492 nm.

La détermination de la molécule d'estradiol à doser est effectuée selon les modalités décrites dans l'exemple 2.

Les résultats obtenus sont présentés respectivement dans les tableaux 7 (tampon PBS 3X) et 8 (tampon PEG) ci-après.

**TABLEAU 7**

| Concentration d'estradiol pg/ml (*) | D.O. 492 nm (**) |
|---|---|
| 0 | 1,875 |
| 0,1 | 1,815 |
| 1 | 1,823 |
| 10 | 1,428 |
| 100 | 0,391 |
| 1000 | 0,053 |

**TABLEAU 8**

| Concentration d'estradiol pg/ml (*) | D.O. 492 nm (**) |
|---|---|
| 0 | 1,859 |
| 0,1 | 1,827 |
| 1 | 1,730 |
| 10 | 1,512 |
| 100 | 0,362 |
| 1000 | 0,058 |

| | |
|---|---|
| * Les échantillons contenant les différentes concentrations d'estradiol sont préparés par addition de quantités connues d'estradiol (17 bêta estradiol commercialisée par la société Sigma sous la référence E 1631) dans du tampon PBS. | |
| ** Chaque point représente la D.O. moyenne de deux valeurs moins la valeur de la fixation non spécifique (lecture 492 nm). | |

Les résultats des gammes d'étalonnage présentés dans les tableaux 7 et 8 montrent une bonne inhibition de la fixation de l'anticorps marqué lorsque la concentration en estradiol augmente.

### EXEMPLE 5 : Utilisation d'un conjugué oligonucléotide-estradiol immobilisé par l'intermédiaire d'un anticorps monoclonal anti-oligonucléotide sur un support solide, pour la détermination d'estradiol par technique immunoenzymatique de type compétition.

L'anticorps monoclonal anti-estradiol utilisé est identique à celui décrit dans l'exemple 2 (BIOMERIEUX ref. 10G6E9H4C1D6).

La courbe de titration est obtenue conformément au protocole décrit dans l'exemple 2, à l'exception de la première étape qui se déroule comme suit : Un anticorps monoclonal anti-oligonucléotide (BIOMERIEUX référence 3H11H10) obtenu par la technique classique décrite par G. Köhler et C. Milstein et purifié à partir de liquides d'ascites, par chromatographie de type échange d'ions ABx (Baker - 72 6900), est immobilisé dans une plaque de microtitration Maxisorb NUNC à raison de 100 µl par puits à une concentration de 10 µg par ml, dans du tampon bicarbonate (NaHCO₃ 0,05 M ; pH 9,6). La plaque est incubée pendant une nuit à 22°C ou bien 1 heure à 37°C.

La plaque est lavée trois fois par 300 µl de tampon PBS-Tween 0,05 % [NaCl 0,15 M ; phosphate de sodium 0,05 M ; pH 7,0 ; Tween 20 à une concentration finale de 0,05 % (Tween : nom commercial)]. Les sites dans la plaque non occupés par l'anticorps monoclonal anti-oligonucléotide sont saturés par l'addition de 100 µl d'une solution de PBS-lait de concentration finale 1% (NaCl 0,15 M ; phosphate de sodium 0,05 M ; pH 7,0 ; lait écrémé Régilait 1 %). La plaque est incubée pendant une heure à 37°C, puis rincée 3 fois par 300 µl de tampon PBS-Tween 0,05 %.

Dans chaque puits sont additionnés 100 µl du conjugué oligonucléotide-haptène G comme décrit dans l'exemple 1 à une concentration de 0,15 µM dans du tampon PBS, pendant 1 heure à 37°C. La plaque est lavée 3 fois par 300 µl de tampon PBS-Tween 0,05 %.

Le reste de l'expérimentation se déroule conformément au protocole décrit dans l'exemple 2.

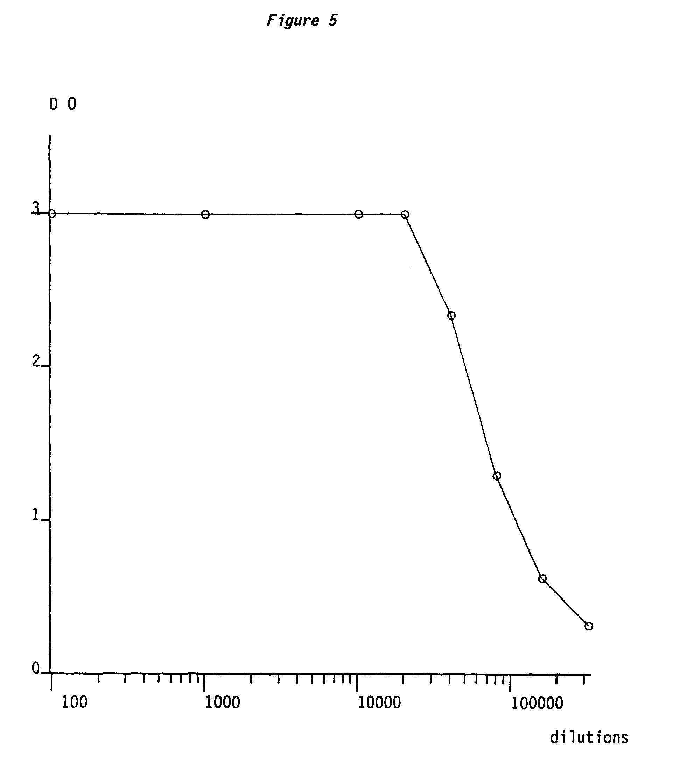

La courbe de titrage obtenue est représentée dans la figure 5 annexée. L'anticorps marqué, qui sera utilisé ultérieurement, est choisi à une dilution déterminée correspondant à une activité enzymatique non saturante, telle que donnant une D.O. de 1,5 à 492 nm.

La détermination de la molécule d'estradiol à doser est effectuée selon les modalités décrites dans l'exemple 2.

Les résultats obtenus sont présentés dans le tableau 9 ci-après.

**TABLEAU 9**

| Concentration d'estradiol pg/ml (*) | D.O. 492 nm (**) |
|---|---|
| 0 | 2,084 |
| 0,1 | 2,028 |
| 1 | 2,061 |
| 10 | 1,810 |
| 100 | 0,452 |
| 1000 | 0,056 |

| | |
|---|---|
| * Les échantillons contenant les différentes concentrations d'estradiol sont préparés par addition de quantités connues d'estradiol (17 bêta estradiol commercialisée par la société Sigma sous la référence E 1631) dans du tampon PBS. | |
| ** Chaque point représente la D.O. moyenne de deux valeurs moins la valeur de la fixation non spécifique (lecture 492 nm). | |

Les résultats de la gamme d'étalonnage présentés dans le tableau 9 montrent une bonne inhibition de la fixation de l'anticorps marqué lorsque la concentration en estradiol augmente.

### EXEMPLE 6 : Utilisation d'un conjugué oligonucléotide-estradiol immobilisé par l'intermédiaire d'un anticorps monoclonal anti-oligonucléotide sur un support solide, pour la détermination d'estradiol par technique immunoenzymatique de type compétition.

Les conditions expérimentales sont identiques à celles décrites dans l'exemple 5, à l'exception de l'utilisation du conjugué oligonucléotide-haptène H et d'un anticorps dirigé contre l'oligonucléotide 1803 (BIO MERIEUX Réf. 3H11H10).

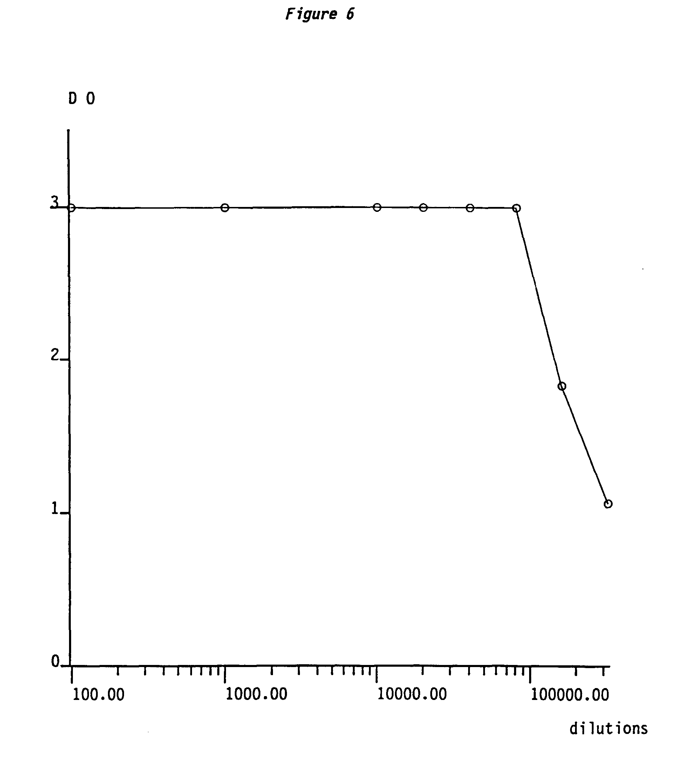

La courbe de titrage obtenue est représentée dans la figure 6 annexée. L'anticorps marqué, qui sera utilisé ultérieurement, est choisi à une dilution déterminée correspondant à une activité enzymatique non saturante, telle que donnant une D.O. de 1,5 à 492 nm.

La détermination de la molécule d'estradiol à doser est effectuée selon les modalités décrites dans l'exemple 2.

Les résultats obtenus sont présentés dans le tableau 10 ci-après.

**TABLEAU 10**

| Concentration d'estradiol pg/ml(*) | D.O. 492 nm (**) |
|---|---|
| 0 | 0,973 |
| 1 | 0,934 |
| 10 | 0,628 |
| 100 | 0,110 |
| 1000 | 0,017 |

| | |
|---|---|
| * Les échantillons contenant les différentes concentrations d'estradiol sont préparés par addition de quantités connues d'estradiol (17-bêta-estradiol commercialisée par la société Sigma sous la référence E 1631) dans du tampon PBS. | |
| ** Chaque point représente la D.O. moyenne de deux valeurs moins la valeur de la fixation non spécifique (lecture 492 nm). | |

Les résultats de la gamme d'étalonnage présentés dans le tableau 10 montrent une bonne inhibition de la fixation de l'anticorps marqué lorsque la concentration en estradiol augmente.

### LISTE DES SEQUENCES

SEQ ID N° 1: ACTAAAAACT AGTAATGCAA AG Longueur: 22
   Type : acide nucléique
   Nombre de brins : simple
   Configuration : linéaire
   Source : synthèse chimique
   Caractéristique additionnelle : bras alkylamine (produit référencé 1 dans le tableau 1) à l'extrémité 5'
   Nom d'usage : 57
SEQ ID N° 2 : ATGTCACGAG CAATTAAGCG Longueur: 20
   Type : acide nucléique
   Nombre de brins : simple
   Configuration : linéaire
   Source : synthèse chimique
   Caractéristique additionnelle : bras dérivé d'aminopropanediol (produit référencé 3 dans le tableau 1) à l'extrémité 3'.
   Nom d'usage : 319
SEQ ID N° 3 : ACTAAAAACT AGNAATGCAA AG Longueur : 22
   Type : acide nucléique
   Nombre de brins : simple
   Configuration : linéaire
   Source : synthèse chimique
   Caractéristique additionnelle : N représente un nucléoside thymidine (produit référencé 2 dans le tableau 1) ayant un bras alkylamine en position 5.
   Nom d'usage : 1257
SEQ ID N° 4 : ACCCCGAGAT TTACGTTATG T Longueur : 21
   Type : acide nucléique
   Nombre de brins :simple
   Configuration : linéaire
   Source : synthèse chimique
   Caractéristiques additionnelles : est constitué de nucléosides d'anomérie alpha et porte un bras alkylamine (produit référencé 1 dans le tableau 1) à l'extrémité 5'.
   Nom d'usage : 196
SEQ ID N° 5 : TTTTTTTTTT TTTTTTTTTT Longueur: 20
   Type : acide nucléique
   Nombre de brins : simple
   Configuration : linéaire
   Source : synthèse chimique
   Caractéristique additionnelle : est constitué de nucléosides d'anomérie alpha et porte un bras dérivé d'aminopropanediol (produit référencé 3 dans le tableau 1) à l'extrémité 3'.
   Nom d'usage : 1803

## Revendications

1. Dispositif pour le dosage d'un haptène ou d'anticorps anti-haptène, comprenant un support solide et un réactif lié de façon covalente à un ligand pour former un conjugué permettant d'immobiliser ledit réactif sur ledit support solide par l'intermédiaire dudit ligand, ledit réactif étant reconnu par des anticorps capables de reconnaître ledit haptène, caractérisé par le fait que ledit ligand est un fragment d'acide nucléique.

2. Dispositif selon la revendication 1, caractérisé par le fait que ledit conjugué est immobilisé par adsorption passive dudit fragment sur le support solide.

3. Dispositif selon la revendication 1, caractérisé par le fait que ledit conjugué est immobilisé par couplage covalent dudit fragment sur le support solide.

4. Dispositif selon la revendication 1, caractérisé par le fait que ledit conjugué est immobilisé sur le support solide par hybridation avec un second fragment d'acide nucléique capable de s'hybrider avec ledit fragment d'acide nucléique dudit conjugué, ledit second fragment d'acide nucléique étant immobilisé sur le support.

5. Dispositif selon la revendication 1, caractérisé par le fait que ledit conjugué est immobilisé sur le support par l'intermédiaire d'un anticorps ou d'un fragment d'anticorps dirigé contre ledit fragment d'acide nucléique dudit conjugué, ledit anticorps étant immobilisé sur ledit support solide.

6. Dispositif selon la revendication 1, caractérisé par le fait qu'il comprend une protéine immobilisée sur le support, ladite protéine étant capable de se lier avec ledit fragment.

7. Dispositif selon l'une quelconque des revendications précédentes, caractérisé par le fait que le fragment d'acide nucléique est choisi parmi un fragment d'ADN, un fragment d'ARN, et un fragment d'ADN ou d'ARN modifié.

8. Dispositif selon la revendication précédente, caractérisé par le fait que ledit fragment d'acide nucléique est un désoxyribonucléotide, un ribonucléotide, un désoxyribonucléoside ou un ribonucléoside.

9. Dispositif selon la revendication 7, caractérisé par le fait que le fragment d'acide nucléique est un oligodésoxyribonucléotide ou un oligoribonucléotide de longueur comprise entre 2 et 100 nucléotides et en particulier entre 10 et 50 nucléotides.

10. Procédé de dosage d'un haptène dans un échantillon liquide, caractérisé par le fait :
- que l'on ajoute audit échantillon une quantité prédéterminée d'anticorps capables de reconnaître ledit haptène ;
- que l'on met en contact l'échantillon avec le dispositif tel que défini dans l'une quelconque des revendications précédentes ;
- que l'on évalue la quantité d'anticorps fixé sur le support solide ;
- et que l'on en déduit, selon les méthodes connues, la quantité d'haptène présente dans l'échantillon.

## Patentansprüche

1. Vorrichtung zur Bestimmung der Dosis eines Haptens oder Antihapten-Antikörpers, umfassend einen festen Träger und ein Reagenz, welches kovalent mit einem Liganden zur Bildung eines Konjugats verbunden ist, das die Immobilisierung des Reagenzes auf dem festen Träger über Vermittlung des Liganden gestattet, wobei das Reagenz von den Antikörpern erkannt wird, die das Hapten erkennen können, dadurch gekennzeichnet, daß es sich bei dem Liganden um ein Nukleinsäurefragment handelt.

2. Vorrichtung gemäß Anspruch 1, dadurch gekennzeichnet, daß das Konjugat mittels passiver Adsorption des Fragments auf dem festen Träger immobilisiert ist.

3. Vorrichtung gemäß Anspruch 1, dadurch gekennzeichnet, daß das Konjugat über kovalente Kupplung des Fragments auf dem festen Träger immobilisiert ist.

4. Vorrichtung gemäß Anspruch 1, dadurch gekennzeichnet, daß das Konjugat auf dem festen Träger über Hybridisierung mit einem zweiten Nucleinsäurefragment immobilisiert ist, welches mit dem konjugierten Nucleinsäurefragment hybridisieren kann, wobei das zweite Nucleinsäurefragment auf dem Träger immobilisiert ist.

5. Vorrichtung gemäß Anspruch 1, dadurch gekennzeichnet, daß das Konjugat auf dem Träger über Vermittlung eines Antikörpers oder eines Antikörperfragments immobilisiert ist, welcher bzw. welches gegen das konjugierte Nucleinsäurefragment gerichtet ist, wobei dieser Antikörper auf dem festen Träger immobilisiert ist.

6. Vorrichtung gemäß Anspruch 1, dadurch gekennzeichnet, daß sie ein auf dem Träger immobilisiertes Protein aufweist, wobei das Protein an das Fragment binden kann.

7. Vorrichtung gemäß einem der vorstehenden Ansprüche, dadurch gekennzeichnet, daß das Nucleinsäurefragment ausgewählt ist unter einem DNS-Fragment, einem RNS-Fragment und einem modfizierten DNS- oder RNS-Fragment.

8. Vorrichtung gemäß dem vorstehenden Anspruch, dadurch gekennzeichnet, daß das Nucleinsäurefragment ein Desoxyribonucleotid, ein Ribonucleotid, ein Desoxyribonucleosid oder ein Ribonucleosid ist.

9. Vorrichtung gemäß Anspruch 7, dadurch gekennzeichnet, daß das Nucleinsäurefragment ein Oligodesoxiribonucleotid oder ein Oligoribonucleotid mit einer Länge zwischen 2 und 100 Nucleotiden und insbesondere zwischen 10 und 50 Nucleotiden darstellt.

10. Verfahren zur Bestimmung der Dosis eines Haptens in einer flüssigen Probe, dadurch gekennzeichnet, daß man
- der Probe eine vorbestimmte Menge eines Antikörpers zusetzt, der das Hapten erkennen kann;
- die Probe mit einer Vorrichtung, definiert gemäß einem der vorstehenden Ansprüche, in Kontakt bringt;
- die auf dem festen Träger fixierte Menge des Antikörpers bestimmt und
- gemäß bekannter Verfahren die in der Probe vorhandene Menge an Hapten ableitet.

## Claims

1. Hapten or anti-hapten antibody assay device comprising a solid support and a reagent covalently linked to a ligand to form a conjugate which makes it possible to immobilize the said reagent onto the said solid support by means of the said ligand, the said reagent being recognized by antibodies capable of recognizing the said hapten, characterized by the fact that the said ligand is a nucleic acid fragment.

2. Device according to Claim 1, characterized by the fact that the said conjugate is immobilized by passive adsorption of the said fragment onto the solid support.

3. Device according to Claim 1, characterized by the fact that the said conjugate is immobilized by covalent coupling of the said fragment onto the solid support.

4. Device according to Claim 1, characterized by the fact that the said conjugate is immobilized onto the solid support by hybridization with a second nucleic acid fragment capable of hybridizing with the said nucleic acid fragment of the said conjugate, the said second nucleic acid fragment being immobilized on the support.

5. Device according to Claim 1, characterized by the fact that the said conjugate is immobilized on the support by means of an antibody or an antibody fragment directed against the said nucleic acid fragment of the said conjugate, the said antibody being immobilized on the said solid support.

6. Device according to Claim 1, characterized by the fact that it comprises a protein immobilized on the support, the said protein being capable of binding with the said fragment.

7. Device according to any one of the preceding claims, characterized by the fact that the nucleic acid fragment is chosen from a DNA fragment, an RNA fragment and a modified DNA or RNA fragment.

8. Device according to the preceding claim, characterized by the fact that the said nucleic acid fragment is a deoxyribonucleotide, a ribonucleotide, a deoxyribonucleoside or a ribonucleoside.

9. Device according to Claim 7, characterized by the fact that the nucleic acid fragment is an oligodeoxyribonucleotide or an oligoribonucleotide with a length of between 2 and 100 nucleotides and in particular between 10 and 50 nucleotides.

10. Process for assaying a hapten in a liquid sample characterized by the fact that:
- a predetermined quantity of antibodies capable of recognizing the said hapten is added to the said sample;
- the sample is brought into contact with the device as defined in any one of the preceding claims;
- the quantity of antibody attached to the solid support is evaluated;
- and the quantity of hapten present in the sample is deduced therefrom according to known methods.
